# EUROPEAN PATENT SPECIFICATION

(11) **EP 3 455 002 B1**
(45) Date of publication and mention of the grant of the patent: **29.07.2020**
(21) Application number: 17730890.5
(22) Date of filing: 08.05.2017
(51) Int. Cl.: B08B 3/04

(54) **CLEANING SYSTEM AND CLEANING METHOD FOR A STYLUS OF AN INOCULATION APPARATUS**
REINIGUNGSSYSTEM UND REINIGUNGSMETHODE FÜR EINE NADEL EINER IMPFVORRICHTUNG
SYSTÈME ET PROCÉDÉ DE NETTOYAGE POUR UNE POINTE D'UN APPAREIL D'INOCULATION

(30) Priority: 08.05.2016 GB 201608009
(43) Date of publication of application: 20.03.2019
(73) Proprietor: Don Whitley Scientific Limited, Bingley West Yorkshire BD16 2NH (GB)
(72) Inventor: KITSELL, Evan, Bingley West Yorkshire, BD16 2NH (GB); BOAST, David, Bingley West Yorkshire, BD16 2NH (GB)
(74) Representative: Murgitroyd & Company
(86) International application number: PCT/GB2017/051274
(87) International publication number: WO 2017/194919

(56) References cited:
- EP-A1- 0 434 994
- EP-A1- 0 648 548
- FR-A1- 2 543 978
- JP-U- S53 150 063
- US-A1- 2012 020 849

## Description

### FIELD OF THE INVENTION

The present invention relates to an automated cleaning system and cleaning method for a stylus of an inoculation apparatus.

### BACKGROUND OF THE INVENTION

During microbiological testing an inoculation apparatus is commonly used to deposit a test sample on a substrate. The inoculation apparatus typically deposits the test sample on the substrate in a predetermined pattern, such as a spiral pattern. As a result, an inoculation apparatus is sometimes referred to as a spiral plater.

An inoculating apparatus conventionally uses a stylus to collect and deposit the test sample on the substrate. To prevent contamination between test samples, the stylus is cleaned after each deposition.

One known way of cleaning the stylus involves dipping the stylus in a washing fluid. However, it has been found that this one-step washing process may not sufficiently clean the stylus. Moreover, any washing fluid remaining on the surface of the stylus may be transferred to next test sample. Conventional cleaning processes also require an operator to regularly top-up the washing fluid. However, manual replenishing is not reliable and human-error may lead to an undersupply or oversupply of washing fluid.

EP 0648548 A1, EP 0434994 A1 and US 2012/0020849 A1 disclose cleaning systems suitable for cleaning a stylus of an inoculation system each comprising a first trough to hold a first washing fluid and comprising a weir and a second trough to hold a second washing fluid and comprising a weir. The first and second trough are being arranged in a cascading series , so that the washing liquid from the first trough overflows in the second trough.

### SUMMARY OF INVENTION

The present invention seeks to address and/or ameliorate the one or more problems described above. The present invention seeks to provide an improved cleaning system and cleaning method for a stylus of an inoculation apparatus.

A first aspect of invention relates to an automated cleaning system for cleaning a stylus of an inoculation apparatus.

The stylus of the inoculation system may be any suitable device for depositing a test sample on a substrate. The stylus may, for example, comprise a syringe, pipette, nozzle or loop.

The automated cleaning system comprises a bath with a plurality of washing troughs in which a stylus can be immersed in washing fluid. As a result, the bath provides multiple and sequential washing steps.

Each washing trough comprises a weir configured to define a washing fluid level and thereby automatically control the level of washing fluid in the washing trough. As a result, the washing fluid in each washing trough has a predetermined maximum level and cannot be exceeded. By using weirs to define fluid levels in the washing troughs, the volume of washing fluid required to fill each trough to the requisite fluid level is known and no complex regulation or measuring of washing fluid is required. The weirs of each washing trough are configured such that, when each washing trough is filled with washing fluid to the predetermined washing fluid level, the level of washing fluid in each washing trough is sufficient to allow the stylus to be immersed in the washing fluid to a desired depth so as to achieve a desired washing effect.

The bath comprises:
a first washing trough with a first weir configured to define a predetermined first washing fluid level; and
a second washing trough with a second weir configured to define a predetermined second washing fluid level, wherein the second washing fluid level is higher than the first washing fluid level; wherein the weirs are configured to prevent the overflow of washing fluid between the washing troughs.

Thus, during use, when the washing troughs are filled with washing fluid to the predetermined fluid levels, the second washing trough has a higher level of washing fluid than the first washing trough. By providing the higher level of washing fluid in the second washing trough to the first washing trough, the stylus is thereby immersible to a greater depth in the second washing fluid than in the first washing fluid. Immersing the stylus to a greater depth in the second washing fluid, advantageously enhances the washing of the stylus and reduces the risk of the first washing fluid remaining on the stylus. For example, if the washing fluid in the first trough comprises a sanitizer and the washing fluid in the second trough comprises a rinser then, by immersing the stylus to a greater depth in the rinser than the sanitizer, the rinsing of the stylus is improved and the transfer of residual sanitizer is limited.

To further enhance the washing of the stylus, the bath may comprise a third washing trough with a third weir configured to define a predetermined third washing fluid level, wherein the third washing fluid level is higher than the first fluid level; and wherein the third weir is configured to prevent overflow of washing fluid between the washing troughs. It follows that the stylus is thereby immersible to a greater depth in the third washing fluid than in the first washing fluid. The third washing fluid may, for example, comprise a rinser to aid the rinsing of the stylus following sanitizing in the first washing trough.

The washing troughs may have any suitable shape and size to hold washing fluid. The washing troughs may have a generally cuboid shape in the form of narrow, elongate recesses. As a result, the surface area of the washing fluids is limited and so the evaporation of the washing fluids is limited. To help provide a compact bath design, the washing troughs are preferably arranged in parallel. To further reduce the size of the bath, the washing troughs may be integrally formed as part of a bath body. Alternatively, the washing troughs may be separately formed. The separately formed washing troughs may comprise coupling means configured to couple the washing troughs together in a sequential arrangement.

The bath may be mountable on a working platform of the inoculation apparatus. The bath may be mountable on the working platform adjacent or convenient to a substrate table so as to limit the movement of the stylus between a substrate and the bath after depositing a test sample on the substrate. Preferably, the bath may be removably mountable on the inoculation apparatus to allow for the removal, cleaning, refitting or replacement of the bath. The narrow configuration of the bath may allow for the length of the working platform to be shortened, and thereby the overall size of the inoculation apparatus reduced.

The automated cleaning system may comprise drive means to move the stylus sequentially between the washing troughs so as to allow the stylus to be initially washed in the first washing trough and subsequently washed in the second washing trough, and optionally washed in the third washing trough. To regulate the position of the stylus in each trough, the drive means may be configured to insert the stylus in each trough to a predetermined distance from the bottom of each trough. The drive means may comprise an arm configured to translate and/or rotate the stylus to a desired position.

The automated cleaning system may comprise washing fluid supply means to supply washing fluid to the troughs of the bath. The supply means may be configured to prime the bath so as to fill each trough with washing fluid to the predetermined fluid levels. To replace any washing fluid consumed during the washing process, the supply means may also be configured to refill each trough with washing fluid to the predetermined fluid levels by adding a replenishing volume of washing fluid. To help limit the contamination of washing fluid in each washing trough, refresh and maintain the efficacy of the washing fluid, the replenishing volume of washing fluid added to each washing trough is preferably greater than the volume of washing fluid consumed during the wash cycle. Advantageously, since the volume of washing fluid consumed during the wash cycle is known and the requisite fluid level in each trough is pre-set by the weirs the replenishing volume of washing flushing can be predetermined.

The washing fluid supply means may comprise a reserve of washing fluid and a spout in fluid communication with the reserve. To supply each trough separately, the washing fluid supply means may comprise:
a reserve of first washing fluid, a first supply pump and a first spout to direct first washing fluid into the first washing trough; and
a reserve of second washing fluid, a second supply pump and a second spout to direct second washing fluid into the second washing trough.

If the bath comprises a third washing trough, the washing fluid means may comprise a reserve of third washing fluid, a third supply pump and a third spout to direct third fluid into the third washing trough.

The first washing fluid preferably comprises a sanitizer to sterilise the stylus. The sanitizer may comprise any suitable sanitizer, for example an alcohol or a chlorine-based disinfectant. The first washing fluid may additionally comprise a detergent to aid the cleaning of the stylus and removal of test sample from the stylus.

The second washing fluid preferably comprises a rinser to rinse the stylus following the sterilizing of the stylus by the first washing fluid. The rinser may comprise any suitable rinser, for example deionised water or sterilised water. The second washing fluid may further comprise a detergent to aid the removal of test sample and sanitizer from the stylus.

If the bath comprises a third washing trough, the third washing fluid preferably comprises a rinser to repeat rinse the stylus. The third washing fluid may comprise any suitable rinser and the third washing fluid and second washing fluid may comprise the same rinser. The third washing fluid may comprise a detergent.

The spouts may be configured in spaced relation to the respective washing troughs. For example, the spouts may be arranged above the respective troughs by a predetermined height. By forming the non-continuous path, the back flow of washing fluid from the washing troughs and thereby contamination of the spouts is restricted.

The automated cleaning system may comprise an overflow trough configured to collect any excess washing fluid overflowing from the washing troughs. The overflow trough is preferably arranged adjacent to weirs of the washing troughs to receive any excess washing fluid overflowing the weirs. Washing fluid may overflow the weirs into the overflow trough during the filling of the troughs, immersion of stylus and/or replenishing of washing fluid.

The overflow trough may be integrally formed with the washing troughs of the bath. Alternatively, the overflow trough may be separately formed. The separately formed overflow trough may comprise coupling means configured to couple the overflow trough to at least one washing trough.

The automated cleaning system may comprise drain means to drain the overflow trough. The draining means may comprise a drain vessel in fluid communication with the overflow trough. The drain vessel may be configured to allow the overflow fluid to drain from the overflow trough to the drain vessel under gravity. Optionally, the drain means may comprise a draining pump to withdraw overflow fluid from the overflow trough into the drain vessel when desired.

If the stylus comprises an internal bore and/or chamber then internal surfaces of the stylus will require washing to restrict contamination. Hence, the automated cleaning system may comprise flushing means to flush through the stylus with washing fluid. The flushing means is configured to flush the stylus when the stylus is immersed in the washing fluid of at least one of the washing troughs and by drawing a volume of the washing fluid through the stylus. The flushing means comprises a flushing vessel in fluid communication with the stylus and a flushing vacuum pump to draw a predetermined volume of washing fluid, from the washing fluid in which the stylus is immersed, through the stylus and into the flushing vessel. The stylus is preferably flushed with each washing fluid as the stylus moves sequentially from trough to trough so that the washing of the internal surfaces of the stylus is consistent with the washing of the external surfaces of the stylus. Advantageously, the gap spaced between the bottom of the trough and the immersed stylus allows for particulates to remain settled at the bottom of trough during the flushing process.

The automated cleaning system may comprise control means to automate the cleaning process. The control means may be configured to control the operation of the drive means so as to automate the moving of the stylus during the washing process. The control means may be configured to control the operation of the washing fluid supply means so as to automate the filling and/or replenishing of washing fluid in each washing trough. For example, the control means may automatically activate the replenishing the washing fluid in each trough after identifying the drive means has withdrawn the stylus from the last washing trough. The control means may be configured to control the operation of the drain means to automate the draining of the overflow trough. The control means may be configured to operate the flushing means to automate the flushing process. The control means may comprise an operator device to allow for operator input in the operation of the automated cleaning system and/or operator warnings.

The automated cleaning system may comprise a cover arrangeable to protect the bath from air-borne contaminants. As a result, the efficacy of the washing fluid is enhanced. The cover may comprises a canopy spaced from the bath by a predetermined distance and shaped to restrict air flow to the bath. For example, the cover may comprise a canopy arranged a predetermined distance above the bath and configured to guide air flow away from the bath. If the bath is mountable on the workstation platform of the inoculation device then the cover may be configured to extend at least substantially along the length of the workstation platform.

A second aspect of the invention relates to an automated method of cleaning a stylus of an inoculation apparatus comprising:
filling a first washing trough with a first washing fluid to a first fluid level defined by a first weir;
filling a second washing trough with a second washing fluid to a second fluid level defined by a second weir, wherein the second fluid level is higher than the first fluid level; and optionally
filling a third washing trough with a third washing fluid to a third fluid level defined by a third weir, wherein the third fluid level is higher than the first fluid level. In this automated method, the weirs are configured to prevent overflow between the washing troughs.

The method further comprising:
immersing a stylus at a first depth in a first washing fluid of the first washing trough;
withdrawing the stylus from the first washing fluid of the first washing trough and immersing a stylus at a second depth in a second washing fluid of the second washing trough, wherein the second depth is greater than the first depth; and optionally
withdrawing the stylus from the second washing fluid of the second washing trough and immersing the stylus at a third depth in a third washing fluid of the third washing trough, wherein the third depth is greater than the first depth.

The method further comprising:
when the stylus is immersed in the first washing fluid of the first washing trough, drawing a predetermined flushing volume of first washing fluid through the stylus; and/or
when the stylus is immersed in the second washing fluid of the second washing trough, drawing a predetermined flushing volume of second washing fluid through the stylus; and/or optionally
when the stylus is immersed in the third washing fluid of the third washing trough, drawing a predetermined flushing volume of third washing fluid through the stylus

The method further comprising
refilling the first washing trough to the first fluid level with a predetermined replenishing volume of first washing fluid, wherein the replenishing volume is greater than the first washing fluid consumed during washing in the first washing trough;
refilling the second washing trough to the second fluid level with a predetermined replenishing volume of second washing fluid, wherein the replenishing volume is greater than the washing fluid consumed during the washing in the second washing trough; and optionally
refilling the third washing trough to the third fluid level with a predetermined replenishing volume of third washing fluid, wherein the replenishing volume is greater than the washing fluid consumed during the washing in the third washing trough.

A third aspect of the invention relates to an inoculation apparatus comprising:
a stylus for depositing a test sample on a substrate;
an automated cleaning system for cleaning the stylus after each deposition according to the first aspect of the invention.

### BRIEF DESCRIPTION OF THE DRAWINGS

For a better understanding of the present invention and to show how it may be carried into effect, reference will now be made, by way of example only, to the accompanying drawings, in which:
Figure 1 is a perspective view of an embodiment of a bath of an automated cleaning system according to the present invention;
Figure 2 is a cross-sectional view showing the weirs of the embodiment of the bath of Figure 1;
Figure 3 is a cross-sectional view showing the different washing fluid levels of the embodiment of the bath of Figure 1;
Figure 4 is a cross-sectional view showing how a stylus can be immersed in the different depths of washing fluid level in the embodiment of the bath of Figure 1;
Figure 5 is a schematic, side view of the embodiment of the bath of Figure 1, washing fluid supply means, drain means and control means of an embodiment of the automated cleaning system according to the present invention; and
Figure 6 is a top view showing the embodiment of the bath of Figure 1 mounted on a working platform of an inoculation apparatus according to the present invention.

### DETAILED DESCRIPTION OF THE EMBODIMENT OF THE INVENTION

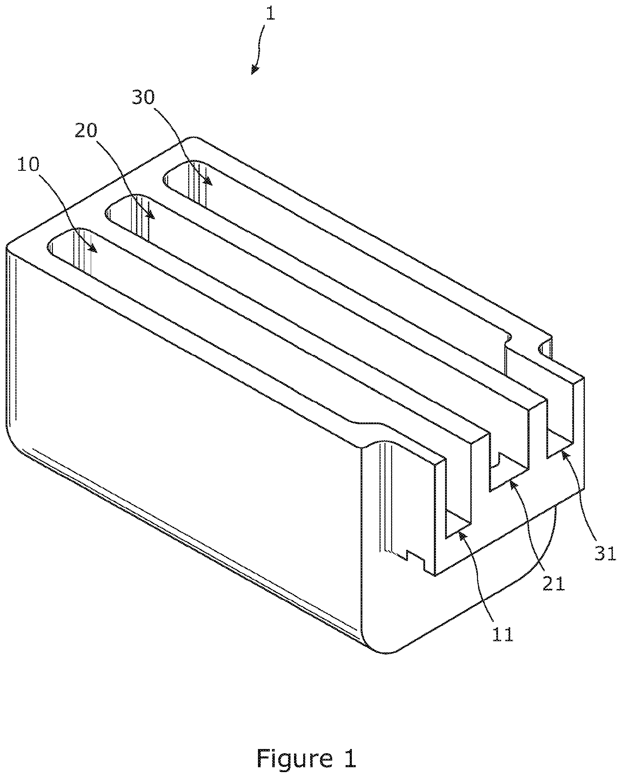

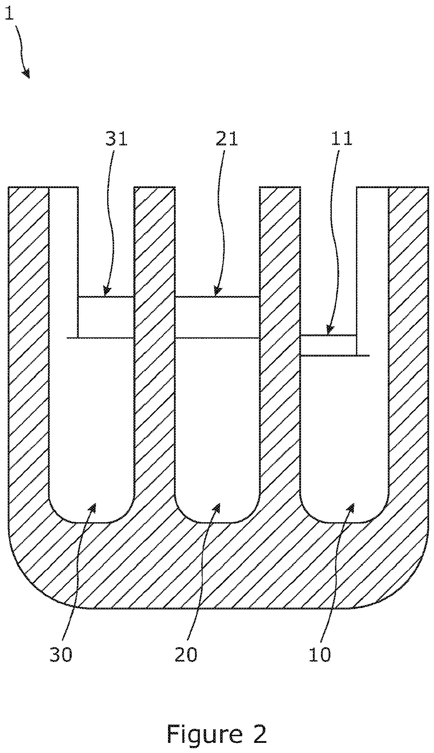

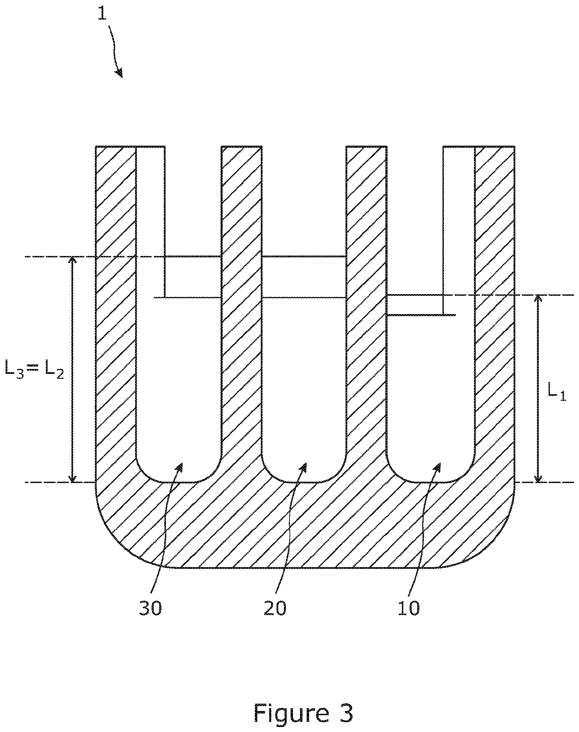

Figures 1 to 6 depict an embodiment of an automated cleaning system for cleaning a stylus (S) in an inoculation system according to the present invention.

The automated cleaning system comprises a bath (1) with a plurality of washing troughs configured to hold washing fluid. As depicted in Figures 1 to 6, this particular embodiment of the bath (1) comprises three washing troughs - a first washing trough (10) for holding a disinfectant, a second washing trough (20) for holding a first rinser and a third washing trough (30) for holding a second rinser.

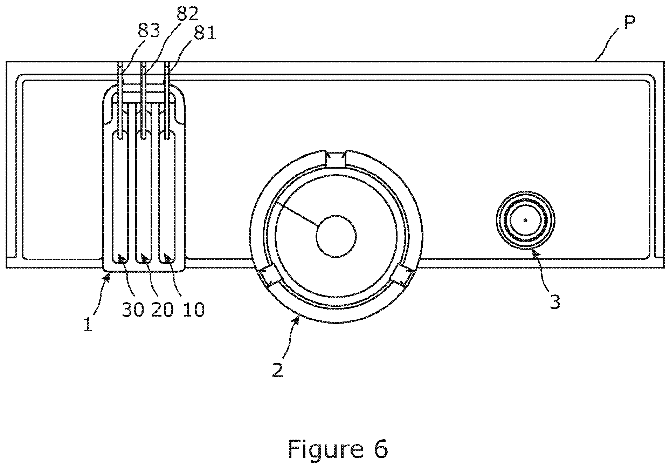

The three washing troughs are cuboid-shaped recesses, integrally formed in the body of the bath. Each trough is approximately 80mm long, approximately 10mm wide and approximately 40mm deep. The troughs are aligned in parallel to allow for sequential access by the stylus. The narrow shape of the troughs helpfully limits the washing fluid surface area so as to restrict the evaporation of the washing fluids. The bath has a compact design due to the configuration of the washing troughs and is approximately 115mm long, approximately 50mm wide and approximately 45mm deep. Figure 6 shows the bath (1) mounted in-situ on the working platform (P) of an inoculation apparatus. The bath is mounted on the working platform (P) so that the longitudinal axis of each trough extends generally perpendicular to the direction of movement of the stylus between the troughs. Due to the miniaturisation of the bath, the length of the working platform, and thereby the overall size of the inoculation apparatus, is reduced. The bath (1) is arranged adjacent to a substrate table (2) so as to minimise the movement of the stylus (S) after deposition between a substrate and the bath. To avoid a direct route of transmission between the bath and the sample, a sample supply (3) is arranged on the opposing side of the substrate table (2) to the bath. The bath is removably mounted on the working platform to allow the bath to be removed, refitted or replaced.

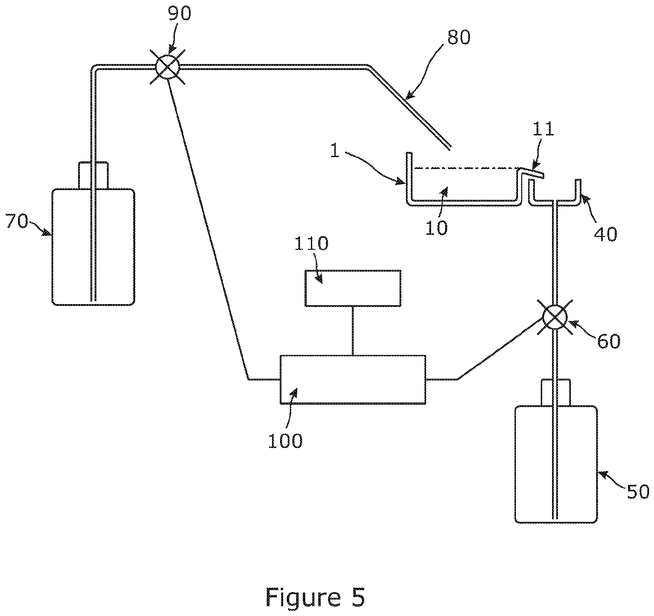

The automated cleaning system comprises washing fluid supply means to supply the washing fluids to the washing troughs of the bath. As shown in Figures 5 and 6, the supply means comprises a reserve of disinfectant (70), a first spout (80/81) to direct disinfectant into the first washing trough (10) and a first supply pump (90) to pump disinfectant from the reserve to the first spout; a reserve of first rinser (not shown), a second spout (82) to direct first rinser into the second washing trough (20) and a second supply pump (not shown); a reserve of second rinser (not shown), a third spout (83) to direct the second rinser into the third washing trough and a third supply pump (not shown). The spouts are spaced from the washing troughs to avoid any backflow of washing fluid. The supply means is configured to prime the bath prior to the washing process so to as to fill each troughs with the requisite washing fluid.

As shown in Figures 1 to 3, each washing trough comprises a weir to automatically control the level of washing fluid that can be held in each trough. The weirs are formed in the rear walls of the washing troughs. In the embodiment depicted, the weirs of the second trough and third trough are configured to define the same washing fluid level (L2 = L3) and this washing fluid level is greater than the washing fluid level for the first trough (L2=L3 > LI). The first washing trough (10) comprises a first weir (11) to limit the level of disinfectant that can be held in the first washing trough to approximately 20mm (L1). The second washing trough (20) comprises a second weir (21) to limit the level of first rinser that can be held in the second washing trough to approximately 25mm (L2). The third washing trough (30) comprises a third weir (31) to limit the level of second rinser that can be held in the third washing trough to approximately 25mm (L3).

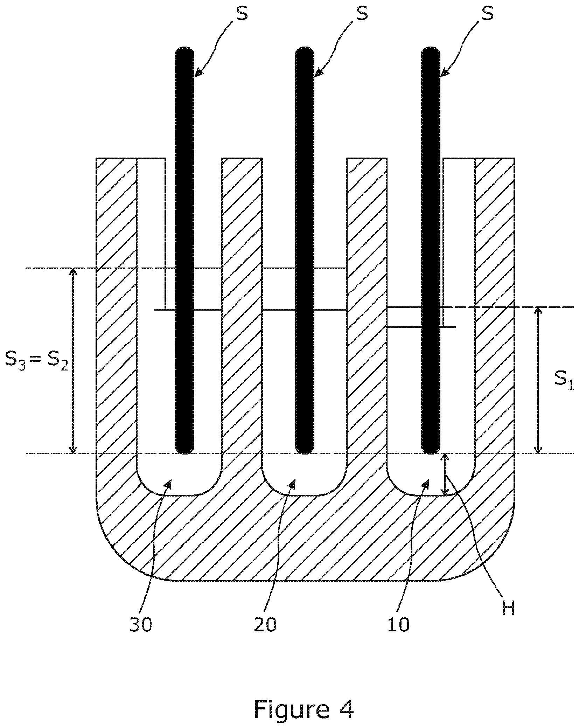

The automated cleaning system comprises a drive means (not shown) to sequentially immerse the stylus in each of the three washing fluids during the washing process. Due to the parallel alignment of the troughs and arrangement of the bath on the inoculation apparatus, the drive means in this embodiment is configured to slide the stylus along a horizontal axis so as to move the stylus sequentially from the first trough to the third trough. The drive means is also configured to raise/lower the stylus along a vertical axis so as to immerse/withdraw the stylus in the washing fluid held in each trough. As can be seen from Figure 4, the drive means is configured to immerse the stylus in the washing liquids by lowering the stylus (S) into each trough to the same predetermined height (H) above the bottom of each trough. Due to the weirs defining different fluid levels in the troughs, the stylus (S) is immersed to a depth of approximately 21mm (S2 = S3) in both the first rinser (S2) and the second rinser (S3) and the stylus is immersed to a depth of approximately 16mm (S1) in the disinfectant. As a result, the stylus is immersed to a greater depth in the first rinser and second rinser than the disinfectant. This rinsing action improves the cleaning of the stylus and improves the removal of any residual disinfectant from the stylus.

Figure 5 shows that the embodiment of the automated cleaning system comprises an overflow trough (40) that is separately formed from the trough and that is arranged adjacent to the weirs of the troughs so as to collect overflowing washing fluid. The overflow trough is drained into a drain vessel (50), when desired, using a drain pump (60).

In this embodiment, the stylus is a suction-type stylus for sucking a volume of test sample from the test sample supply (3) into the stylus prior to deposition on a substrate. To clean the internal surfaces of the stylus, the cleaning means comprises flushing means to flush through the stylus with the disinfectant, first rinser and second rinser. The flushing means comprises a flushing pump (not shown) to draw a predetermined volume of disinfectant, first rinser and second rinser through the stylus and into a flush vessel (not shown), when the stylus is sequentially immersed in washing fluid of the washing troughs.

Since washing fluid is consumed during the washing process, the supply means is also configured to replenish washing fluid to the fluid levels defined by the weirs in each trough. Since the volume of the washing fluid consumed during the washing process is predetermined, the supply means is configured to supply a predetermined replenishing volume of washing fluid to each trough. Preferably, the predetermined replenishing volume is greater than the consumed volume so as to generate an overflow to help refresh and remove any floating contaminates in the washing fluid.

In the embodiment depicted in Figure 5, the automated cleaning system comprises control means 100 to automate the operation of the automated cleaning system. The control means is configured to control the drive means so as to automate the movement of the stylus between the washing troughs, to control the washing fluid supply means so as to automate the supply of washing fluid, to control the drain means so to as to automate the draining of the overflow trough and to control the flushing means so as to automate the flushing of washing fluid through the stylus. The control means preferably comprises an operator device 110 to allow for operator input and/or provide operator warnings.

In the embodiment, the automated cleaning system comprises a canopy (not shown) to guide air flow and any airborne contaminants away from the bath (1), substrate table (2) and sample supply (3) on the working platform (P).

Although the invention has been described above with reference to one or more preferred embodiments, it will be appreciated that various changes or modifications may be made without departing from the scope of the invention as defined in the appended claims.

## Claims

1. An automated cleaning system for cleaning a stylus (S) of an inoculation apparatus comprising a bath (1), the bath (1) comprising:
a first washing trough (10) to hold a first washing fluid and comprising a first weir (11) defining a first washing fluid level (L1); and
a second washing trough (20) to hold a second washing fluid and comprising a second weir (21) defining a second washing fluid level (L2), wherein the second washing fluid level (L2) is higher than the first washing fluid level (L1) and so the stylus (S) is thereby immersible to a greater depth in the second washing fluid than in the first washing fluid;
**characterised in that** the first and second weirs are configured to prevent overflow of washing fluid between the washing troughs.

2. The cleaning system as defined in claim 1, further comprising a third washing trough (30) to hold a third washing fluid and comprising a third weir (31) defining a third washing fluid level (L3), wherein the third washing fluid level (L3) is higher than the first washing fluid level (L1) and so the stylus (S) is thereby immersible to a greater depth in the third washing fluid than in the first washing fluid; and wherein the third weir (31) is configured to prevent overflow of washing fluid between the washing troughs.

3. The cleaning system as defined in claim 1 or 2, wherein the troughs (10, 20, 30) are integrally formed or separately formed;
and/or wherein the bath is configured to be removably mountable on the inoculation apparatus, preferably on a workstation platform of the inoculation apparatus.

4. The cleaning system as defined in any preceding claim, further comprising a drive means configured to sequentially immerse the stylus (S) in the first washing fluid of the first washing trough (10) and subsequently the second washing fluid of the second washing trough (20), and optionally the third washing fluid of the third washing trough (30);
and further optionally wherein the drive means is configured to immerse the stylus by inserting the stylus in the said washing trough, a predetermined distance from the bottom of the said washing trough.

5. The cleaning system defined in any preceding claim, further comprising a flushing means to flush washing fluid through the stylus (S) when the stylus is immersed in the washing fluid of at least one washing through, the flushing means comprising:
a flushing vessel in fluid communication with the stylus; and
a flushing pump configured to draw a predetermined flushing volume of washing fluid through the stylus to the flushing vessel.

6. The cleaning system defined in any preceding claim, further comprising supply means configured to supply washing fluid to each washing trough, the supply means configured:
to fill each washing trough with washing fluid to the respective washing fluid levels prior to immersing the stylus;
to replenish the washing fluid in each washing trough to the respective washing fluid levels following the immersion and withdrawal of the stylus from the washing fluid;
and optionally wherein the supply means is configured to replenish the washing fluid in each washing trough by adding a predetermined replenishing volume of washing fluid to each washing trough, wherein the replenishing volume is greater than the volume of washing fluid consumed from each respective trough during the wash cycle.

7. The cleaning system as defined in claim 6, wherein the supply means comprises:
a reserve (70) of first washing fluid, a first pump (90) and a first spout (80) in fluid communication with the first reserve (70) and arranged in spaced relation above the first washing trough (10);
a reserve of second washing fluid, a second pump and a second spout (82) in fluid communication with the second washing fluid and arranged in spaced relation above the second washing trough (20);
and optionally a reserve of third washing fluid, a third pump and a third spout (83) arranged in spaced relation above the third washing trough.

8. The cleaning system according to any preceding claim, wherein the first washing fluid comprises a sanitizer, the second washing fluid comprises a rinser and optionally the third washing fluid comprises a rinser;
and/or further comprising a cover to guide airflow away from the bath and thereby protect the bath from airborne contamination.

9. The cleaning system according to any preceding claims, further comprising an overflow trough (40) arranged adjacent to the weirs of the washing troughs;
and optionally further comprising a drain means comprising a drain vessel (50), and even further optionally a drain pump (60) to withdraw fluid from the overflow trough and into the drain vessel.

10. The cleaning system according to any preceding claim comprising control means (100) to automatically control the cleaning system;
and optionally wherein the control means (100) comprises an operator device (110) comprising operator input and/or operator warning.

11. An inoculation apparatus comprising a cleaning system as defined in any of claims 1 to 10.

12. An automated method for cleaning a stylus of an inoculation apparatus using a cleaning system as defined in any of claims 1 to 10, the method comprising:
filling a first washing trough (10) with a first washing fluid to a first washing fluid level (L1) defined by a first weir (11);
filling a second washing trough (20) with a second washing fluid to a second washing fluid level (L2) defined by a second weir (21), wherein the second fluid level (L2) is higher than the first fluid level (L1); **characterised in that** the first and second weirs are configured to prevent overflow of washing fluid between the washing troughs.

13. The method according to claim 12, further comprising:
filling a third washing trough (30) with a third washing fluid to a third fluid level (L3) defined by a third weir (31), wherein the third fluid level (L3) is higher than the first fluid level (L1);
wherein the third weir is configured to prevent overflow of fluid between the washing troughs.

14. The method according to claim 12 or 13, further comprising:
immersing a stylus (S) at a first depth in the first washing fluid of the first washing trough (10);
withdrawing the stylus from the first washing fluid of the first washing trough and immersing the stylus at a second depth in the second washing fluid of the second washing trough (20), wherein the second depth is greater than the first depth;
and optionally withdrawing the stylus from the second washing fluid of the second washing trough and immersing the stylus at a third depth in the third washing fluid of the third washing trough (30), wherein the third depth is greater than the first depth; and
optionally further comprising:
when the stylus is immersed in the first washing fluid of the first washing trough (10), drawing a predetermined flushing volume of first washing fluid through the stylus;
and/or when the stylus is immersed in the second washing fluid of the second washing trough (20), drawing a predetermined flushing volume of second washing fluid through the stylus;
and/or optionally when the stylus is immersed in the third washing fluid of the third washing trough (30), drawing a predetermined flushing volume of third washing fluid through the stylus.

15. The method according to claim 14 further comprising:
refilling the first washing trough to the first washing fluid level with a predetermined replenishing volume of first washing fluid, wherein the replenishing volume is greater than the volume of first washing fluid consumed during washing in the first washing trough (10);
refilling the second washing trough to the second washing fluid level with a predetermined replenishing volume of second washing fluid, wherein the replenishing volume is greater than the volume of washing fluid consumed during the washing in the second washing trough (20);
and optionally refilling the third washing trough to the third fluid level with a predetermined replenishing volume of third washing fluid, wherein the replenishing volume is greater than the volume of washing fluid consumed during the washing in the third washing trough (30).

## Patentansprüche

1. Ein automatisches Reinigungssystem zum Reinigen einer Nadel (S) einer Impfvorrichtung, das ein Bad (1) beinhaltet, wobei das Bad (1) Folgendes beinhaltet:
einen ersten Waschtrog (10) zum Halten eines ersten Waschfluids, der einen einen ersten Waschfluidpegel (L1) definierenden ersten Überlauf (11) beinhaltet; und
einen zweiten Waschtrog (20) zum Halten eines zweiten Waschfluids, der einen einen zweiten Waschfluidpegel (L2) definierenden zweiten Überlauf (21) beinhaltet, wobei der zweite Waschfluidpegel (L2) höher als der erste Waschfluidpegel (L1) liegt und die Nadel (S) dadurch so tiefer in das zweite Waschfluid als in das erste Waschfluid eingetaucht werden kann;
**dadurch gekennzeichnet, dass** der erste und zweite Überlauf konfiguriert sind, um ein Überströmen von Waschfluid zwischen den Waschtrögen zu verhindern.

2. Reinigungssystem wie in Anspruch 1 definiert, ferner beinhaltend einen dritten Waschtrog (30) zum Halten eines dritten Waschfluids, der einen einen dritten Waschfluidpegel (L3) definierenden dritten Überlauf (31) beinhaltet, wobei der dritte Waschfluidpegel (L3) höher als der erste Waschfluidpegel (L1) liegt und die Nadel (S) dadurch so tiefer in das dritte Waschfluid als in das erste Waschfluid eingetaucht werden kann; und wobei der dritte Überlauf (31) konfiguriert ist, um ein Überströmen von Waschfluid zwischen den Waschtrögen zu verhindern.

3. Reinigungssystem wie in Anspruch 1 oder 2 definiert, wobei die Tröge (10, 20, 30) integral oder getrennt ausgebildet sind; und/oder
wobei das Bad konfiguriert ist, um abnehmbar auf der Impfvorrichtung angebracht werden zu können, vorzugsweise auf einer Plattform einer Arbeitsstation der Impfvorrichtung.

4. Reinigungssystem wie in einem der vorhergehenden Ansprüche definiert, ferner beinhaltend eine Antriebseinrichtung, die konfiguriert ist, um die Nadel (S) nacheinander in das erste Waschfluid des ersten Waschtrogs (10) und anschließend das zweite Waschfluid des zweiten Waschtrogs (20) und gegebenenfalls das dritte Waschfluid des dritten Waschtrogs (30) einzutauchen;
und wobei ferner gegebenenfalls die Antriebseinrichtung konfiguriert ist, um die Nadel durch Einführen der Nadel in den Waschtrog zu einem vorgegebenen Abstand von dem Boden des Waschtrogs einzutauchen.

5. Reinigungssystem wie in einem der vorhergehenden Ansprüche definiert, ferner beinhaltend eine Spüleinrichtung, um Waschfluid durch die Nadel (S) zu spülen, wenn die Nadel in das Waschfluid mindestens eines Waschtrogs eingetaucht ist, wobei die Spüleinrichtung Folgendes beinhaltet:
ein Spülgefäß in Fluidverbindung mit der Nadel; und
eine Spülpumpe, die konfiguriert ist, um eine vorgegebene Spülmenge von Waschfluid durch die Nadel in das Spülgefäß zu ziehen.

6. Reinigungssystem wie in einem der vorhergehenden Ansprüche definiert, ferner beinhaltend eine Zufuhreinrichtung, die konfiguriert ist, um jedem Waschtrog Waschfluid zuzuführen, wobei die Zufuhreinrichtung für Folgendes konfiguriert ist:
Füllen jedes Waschtrogs mit Waschfluid bis zu den jeweiligen Waschfluidpegeln vor dem Eintauchen der Nadel;
Nachfüllen des Waschfluids in jedem Waschtrog bis zu den jeweiligen Waschfluidpegeln im Anschluss an das Eintauchen und die Entnahme der Nadel aus dem Waschfluid;
und wobei gegebenenfalls die Zufuhreinrichtung konfiguriert ist, um das Waschfluid in jedem Waschtrog durch Zugeben einer vorgegebenen Nachfüllmenge von Waschfluid zu jedem Waschtrog nachzufüllen, wobei die Nachfüllmenge größer als die Menge von Waschfluid ist, die aus jedem jeweiligen Trog während des Waschzyklus verbraucht wurde.

7. Reinigungssystem wie in Anspruch 6 definiert, wobei die Zufuhreinrichtung Folgendes beinhaltet:
einen Vorrat (70) von erstem Waschfluid, eine erste Pumpe (90) und eine in Fluidverbindung mit dem ersten Vorrat (70) stehende erste Tülle (80), die in beabstandeter Beziehung über dem ersten Waschtrog (10) angeordnet ist;
einen Vorrat von zweitem Waschfluid, eine zweite Pumpe und eine in Fluidverbindung mit dem zweiten Waschfluid stehende zweite Tülle (82), die in beabstandeter Beziehung über dem zweiten Waschtrog (20) angeordnet ist;
und gegebenenfalls einen Vorrat von drittem Waschfluid, eine dritte Pumpe und eine dritte Tülle (83), die in beabstandeter Beziehung über dem dritten Waschtrog angeordnet ist.

8. Reinigungssystem gemäß einem der vorhergehenden Ansprüche, wobei das erste Waschfluid einen Entkeimer beinhaltet, das zweite Waschfluid einen Klarspüler beinhaltet und gegebenenfalls das dritte Waschfluid einen Klarspüler beinhaltet; und/oder
ferner eine Abdeckung beinhaltend, um einen Luftstrom von dem Bad wegzuführen und dadurch das Bad vor luftgetragener Verschmutzung zu schützen.

9. Reinigungssystem gemäß einem der vorhergehenden Ansprüche, ferner beinhaltend einen Überströmtrog (40), der an die Überläufe der Waschtröge angrenzend angeordnet ist;
und gegebenenfalls ferner beinhaltend eine Ablaufeinrichtung, die ein Ablaufgefäß (50) beinhaltet,
und noch gegebenenfalls ferner eine Ablaufpumpe (60), um Fluid aus dem Überströmtrog und in das Ablaufgefäß zu entnehmen.

10. Reinigungssystem gemäß einem der vorhergehenden Ansprüche, beinhaltend eine Steuereinrichtung (100), um das Reinigungssystem automatisch zu steuern;
und wobei gegebenenfalls die Steuereinrichtung (100) ein Bedienergerät (110) beinhaltet, das eine Bedienereingabe und/oder eine Bedienerwarnung beinhaltet.

11. Eine Impfvorrichtung, beinhaltend ein Reinigungssystem wie in einem der Ansprüche 1 bis 10 definiert.

12. Ein automatisches Verfahren zum Reinigen einer Nadel einer Impfvorrichtung unter Verwendung eines Reinigungssystems wie in einem der Ansprüche 1 bis 10 definiert, wobei das Verfahren Folgendes beinhaltet:
Füllen eines ersten Waschtrogs (10) mit einem ersten Waschfluid bis zu einem von einem ersten Überlauf (11) definierten ersten Waschfluidpegel (L1);
Füllen eines zweiten Waschtrogs (20) mit einem zweiten Waschfluid bis zu einem von einem zweiten Überlauf (21) definierten zweiten Waschfluidpegel (L2), wobei der zweite Fluidpegel (L2) höher als der erste Fluidpegel (L1) liegt;
**dadurch gekennzeichnet, dass** der erste und zweite Überlauf konfiguriert sind, um ein Überströmen von Waschfluid zwischen den Waschtrögen zu verhindern.

13. Verfahren gemäß Anspruch 12, ferner beinhaltend:
Füllen eines dritten Waschtrogs (30) mit einem dritten Waschfluid bis zu einem von einem dritten Überlauf (31) definierten dritten Fluidpegel (L3), wobei der dritte Fluidpegel (L3) höher als der erste Fluidpegel (L1) liegt;
wobei der dritte Überlauf konfiguriert ist, um ein Überströmen von Fluid zwischen den Waschtrögen zu verhindern.

14. Verfahren gemäß Anspruch 12 oder 13, ferner beinhaltend:
Eintauchen einer Nadel (S) auf eine erste Tiefe in dem ersten Waschfluid des ersten Waschtrogs (10);
Entnehmen der Nadel aus dem ersten Waschfluid des ersten Waschtrogs und
Eintauchen der Nadel auf eine zweite Tiefe in dem zweiten Waschfluid des zweiten Waschtrogs (20), wobei die zweite Tiefe größer als die erste Tiefe ist;
und gegebenenfalls Entnehmen der Nadel aus dem zweiten Waschfluid des zweiten Waschtrogs und Eintauchen der Nadel auf eine dritte Tiefe in dem dritten Waschfluid des dritten Waschtrogs (30), wobei die dritte Tiefe größer als die erste Tiefe ist;
und gegebenenfalls ferner beinhaltend:
wenn die Nadel in dem ersten Waschfluid des ersten Waschtrogs (10) eingetaucht ist, Ziehen einer vorgegebenen Spülmenge von erstem Waschfluid durch die Nadel;
und/oder wenn die Nadel in dem zweiten Waschfluid des zweiten Waschtrogs (20) eingetaucht ist, Ziehen einer vorgegebenen Spülmenge von zweitem Waschfluid durch die Nadel;
und/oder gegebenenfalls wenn die Nadel in dem dritten Waschfluid des dritten Waschtrogs (30) eingetaucht ist, Ziehen einer vorgegebenen Spülmenge von drittem Waschfluid durch die Nadel.

15. Verfahren gemäß Anspruch 14, ferner beinhaltend:
Auffüllen des ersten Waschtrogs bis zu dem ersten Waschfluidpegel mit einer vorgegebenen Nachfüllmenge von erstem Waschfluid, wobei die Nachfüllmenge größer als die Menge von erstem Waschfluid ist, die während des Waschens in dem ersten Waschtrog (10) verbraucht wurde;
Auffüllen des zweiten Waschtrogs bis zu dem zweiten Waschfluidpegel mit einer vorgegebenen Nachfüllmenge von zweitem Waschfluid, wobei die Nachfüllmenge größer als die Menge von Waschfluid ist, die während des Waschens in dem zweiten Waschtrog (20) verbraucht wurde;
und gegebenenfalls Auffüllen des dritten Waschtrogs bis zu dem dritten Fluidpegel mit einer vorgegebenen Nachfüllmenge von drittem Waschfluid, wobei die Nachfüllmenge größer als die Menge von Waschfluid ist, die während des Waschens in dem dritten Waschtrog (30) verbraucht wurde.

## Revendications

1. Un système de nettoyage automatisé pour le nettoyage d'un stylet (S) d'un appareil d'inoculation comprenant un bain (1), le bain (1) comprenant :
un premier bac de lavage (10) destiné à contenir un premier fluide de lavage et comprenant un premier déversoir (11) définissant un premier niveau de fluide de lavage (L1) ; et
un deuxième bac de lavage (20) destiné à contenir un deuxième fluide de lavage et
comprenant un deuxième déversoir (21) définissant un deuxième niveau de fluide de lavage (L2), le deuxième niveau de fluide de lavage (L2) étant plus haut que le premier niveau de fluide de lavage (L1) et de sorte que le stylet (S) puisse dès lors être immergé à une plus grande profondeur dans le deuxième fluide de lavage que dans le premier fluide de lavage ;
**caractérisé en ce que** le premier et le deuxième déversoir sont configurés pour empêcher que du fluide de lavage ne déborde d'un bac de lavage dans l'autre.

2. Le système de nettoyage tel que défini dans la revendication 1, comprenant en sus un troisième bac de lavage (30) destiné à contenir un troisième fluide de lavage et comprenant un troisième déversoir (31) définissant un troisième niveau de fluide de lavage (L3), le troisième niveau de fluide de lavage (L3) étant plus haut que le premier niveau de fluide de lavage (L1) et de sorte que le stylet (S) puisse dès lors être immergé à une plus grande profondeur dans le troisième fluide de lavage que dans le premier fluide de lavage ; et dans lequel le troisième déversoir (31) est configuré pour empêcher que du fluide de lavage ne déborde d'un bac de lavage dans l'autre.

3. Le système de nettoyage tel que défini dans la revendication 1 ou la revendication 2, dans lequel les bacs (10, 20, 30) sont formés de façon solidaire ou séparée ; et/ou dans lequel le bain est configuré pour pouvoir être monté de façon amovible sur l'appareil d'inoculation, de préférence sur une plateforme de station de travail de l'appareil d'inoculation.

4. Le système de nettoyage tel que défini dans n'importe quelle revendication précédente, comprenant en sus un moyen d'entraînement configuré pour immerger le stylet (S) de façon séquentielle dans le premier fluide de lavage du premier bac de lavage (10), puis dans le deuxième fluide de lavage du deuxième bac de lavage (20), et facultativement dans le troisième fluide de lavage du troisième bac de lavage (30) ;
et facultativement encore dans lequel le moyen d'entraînement est configuré pour immerger le stylet en insérant le stylet dans ledit bac de lavage, à une distance prédéterminée du fond dudit bac de lavage.

5. Le système de nettoyage défini dans n'importe quelle revendication précédente, comprenant en sus un moyen de rinçage destiné à faire passer du fluide de lavage à travers le stylet (S) lorsque le stylet est immergé dans le fluide de lavage d'au moins un bac de lavage, le moyen de rinçage comprenant :
un récipient de rinçage en communication fluidique avec le stylet ; et
une pompe de rinçage configurée pour tirer un volume de rinçage prédéterminé de fluide de lavage à travers le stylet jusque dans le récipient de rinçage.

6. Le système de nettoyage défini dans n'importe quelle revendication précédente, comprenant en sus un moyen d'approvisionnement configuré pour approvisionner chaque bac de lavage en fluide de lavage, le moyen d'approvisionnement étant configuré pour :
remplir chaque bac de lavage de fluide de lavage jusqu'aux niveaux de fluide de lavage respectifs avant immersion du stylet ;
refaire le plein de fluide de lavage dans chaque bac de lavage jusqu'aux niveaux de fluide de lavage respectifs après immersion et retrait du stylet du fluide de lavage ;
et facultativement dans lequel le moyen d'approvisionnement est configuré pour refaire le plein de fluide de lavage dans chaque bac de lavage en ajoutant un volume de complément de plein prédéterminé de fluide de lavage dans chaque bac de lavage, le volume de complément de plein étant plus grand que le volume de fluide de lavage consommé dans chaque bac respectif durant le cycle de lavage.

7. Le système de nettoyage tel que défini dans la revendication 6, dans lequel le moyen d'approvisionnement comprend :
une réserve (70) de premier fluide de lavage, une première pompe (90) et une première goulotte (80) en communication fluidique avec la première réserve (70) et agencée à l'écart et au-dessus du premier bac de lavage (10) ;
une réserve de deuxième fluide de lavage, une deuxième pompe et une deuxième goulotte (82) en communication fluidique avec le deuxième fluide de lavage et agencée à l'écart et au-dessus du deuxième bac de lavage (20) ;
et facultativement une réserve de troisième fluide de lavage, une troisième pompe et une troisième goulotte (83) agencée à l'écart et au-dessus du troisième bac de lavage.

8. Le système de nettoyage selon n'importe quelle revendication précédente, dans lequel le premier fluide de lavage comprend un assainissant, le deuxième fluide de lavage comprend une formulation rinçante et facultativement le troisième fluide de lavage comprend une formulation rinçante ; et/ou
comprenant en sus un couvercle destiné à détourner du bain un écoulement d'air et par là à protéger le bain d'une contamination en suspension dans l'air.

9. Le système de nettoyage selon n'importe lesquelles des revendications précédentes, comprenant en sus un bac de débordement (40) agencé adjacent aux déversoirs des bacs de lavage ;
et comprenant facultativement en sus un moyen de drainage comprenant un récipient de drainage (50),
et même facultativement encore une pompe de drainage (60) destinée à retirer du fluide du bac de débordement et à l'amener dans le récipient de drainage.

10. Le système de nettoyage selon n'importe quelle revendication précédente comprenant un moyen de commande (100) destiné à commander automatiquement le système de nettoyage ;
et facultativement dans lequel le moyen de commande (100) comprend un dispositif pour opérateur (110) comprenant une entrée pour opérateur et/ou un avertissement pour opérateur.

11. Un appareil d'inoculation comprenant un système de nettoyage tel que défini dans n'importe lesquelles des revendications 1 à 10.

12. Un procédé automatisé pour le nettoyage d'un stylet d'un appareil d'inoculation en utilisant un système de nettoyage tel que défini dans n'importe lesquelles des revendications 1 à 10, le procédé comprenant :
le remplissage d'un premier bac de lavage (10) avec un premier fluide de lavage jusqu'à un premier niveau de fluide de lavage (L1) défini par un premier déversoir (11) ; le remplissage d'un deuxième bac de lavage (20) avec un deuxième fluide de lavage jusqu'à un deuxième niveau de fluide de lavage (L2) défini par un deuxième déversoir (21), dans lequel le deuxième niveau de fluide (L2) est plus haut que le premier niveau de fluide (L1) ;
**caractérisé en ce que** le premier et le deuxième déversoir sont configurés pour empêcher que du fluide de lavage ne déborde d'un bac de lavage dans l'autre.

13. Le procédé selon la revendication 12, comprenant en sus :
le remplissage d'un troisième bac de lavage (30) avec un troisième fluide de lavage jusqu'à un troisième niveau de fluide (L3) défini par un troisième déversoir (31), dans lequel le troisième niveau de fluide (L3) est plus haut que le premier niveau de fluide (L1) ;
dans lequel le troisième déversoir est configuré pour empêcher que du fluide ne déborde d'un bac de lavage dans l'autre.

14. Le procédé selon la revendication 12 ou la revendication 13, comprenant en sus :
l'immersion d'un stylet (S) à une première profondeur dans le premier fluide de lavage du premier bac de lavage (10) ;
le retrait du stylet du premier fluide de lavage du premier bac de lavage et l'immersion du stylet à une deuxième profondeur dans le deuxième fluide de lavage du deuxième bac de lavage (20), la deuxième profondeur étant plus grande que la première profondeur ;
et facultativement le retrait du stylet du deuxième fluide de lavage du deuxième bac de lavage et l'immersion du stylet à une troisième profondeur dans le troisième fluide de lavage du troisième bac de lavage (30), la troisième profondeur étant plus grande que la première profondeur ;
et comprenant facultativement en sus :
lorsque le stylet est immergé dans le premier fluide de lavage du premier bac de lavage (10), tirer un volume de rinçage prédéterminé de premier fluide de lavage à travers le stylet ;
et/ou lorsque le stylet est immergé dans le deuxième fluide de lavage du deuxième bac de lavage (20), tirer un volume de rinçage prédéterminé de deuxième fluide de lavage à travers le stylet ;
et/ou facultativement lorsque le stylet est immergé dans le troisième fluide de lavage du troisième bac de lavage (30), tirer un volume de rinçage prédéterminé de troisième fluide de lavage à travers le stylet.

15. Le procédé selon la revendication 14 comprenant en sus :
le re-remplissage du premier bac de lavage jusqu'au premier niveau de fluide de lavage avec un volume de complément de plein prédéterminé de premier fluide de lavage, le volume de complément de plein étant plus grand que le volume de premier fluide de lavage consommé durant le lavage dans le premier bac de lavage (10) ;
le re-remplissage du deuxième bac de lavage jusqu'au deuxième niveau de fluide de lavage avec un volume de complément de plein prédéterminé de deuxième fluide de lavage, le volume de complément de plein étant plus grand que le volume de fluide de lavage consommé durant le lavage dans le deuxième bac de lavage (20) ;
et facultativement le re-remplissage du troisième bac de lavage jusqu'au troisième niveau de fluide avec un volume de complément de plein prédéterminé de troisième fluide de lavage, le volume de complément de plein étant plus grand que le volume de fluide de lavage consommé durant le lavage dans le troisième bac de lavage (30).
